# FASCICULE DE BREVET EUROPEEN

(11) **EP 1 068 310 B1**
(45) Date de publication et mention de la délivrance du brevet: **02.04.2008**
(21) Numéro de dépôt: 99903787.2
(22) Date de dépôt: 19.02.1999
(51) Int. Cl.: C12N 15/11, C12Q 1/68, C07K 14/47

(54) **METHODE DE CRIBLAGE DE COMPOSES CAPABLES D'INHIBER LA FIXATION ENTRE LE FACTEUR DE TRANSCRIPTION STAT1 ET LE FACTEUR DE TRANSCRIPTION USF1**
VERFAHREN ZUM AUFFINDEN VON VERBINDUNGEN, DIE DIE BINDUNG ZWISCHEN DEN TRANSKRIPTIONSFAKTOREN STAT1 UND USF1 HEMMEN
METHOD FOR SCREENING COMPOUNDS CAPABLE OF INHIBITING FIXING BETWEEN THE TRANSCRIPTION FACTOR OF STAT1 AND THE TRANSCRIPTION FACTOR OF USF1

(30) Priorité: 19.02.1998 FR 9802025
(43) Date de publication de la demande: 17.01.2001
(73) Titulaire: Novimmune SA, 1211 Genève (CH)
(72) Inventeur: MACH, Bernard, CH-1292 Chambésy, Genève (CH)
(74) Mandataire: Almond-Martin, Carol
(86) Numéro de dépôt international: PCT/FR1999/000376
(87) Numéro de publication internationale: WO 1999/042571

(56) Documents cités:
- EP-A- 0 874 049
- WO-A-95/28482
- WO-A-95/28492
- WO-A-96/15265
- MUHLETHALER-MOTTET A ET AL: "Activation of the MHC class II transactivator CIITA by interferon-gamma requires cooperative interaction between Stat1 and USF -1." IMMUNITY, (1998 FEB) 8 (2) 157-66., XP002083358
- MUHLETHALER-MOTTET A ET AL: "EXPRESSION OF MHC CLASS II MOLECULES IN DIFFERENT CELLULAR AND FUNCTIONAL COMPARTMENTS IS CONTROLLED BY DIFFERENTIAL USAGE OF MULTIPLE PROMOTORS OF THE TRANSACTIVATOR CIITA" EMBO JOURNAL, vol. 16, no. 10, 15 mai 1997 (1997-05-15), pages 2851-2860, XP002051561 cité dans la demande
- PISKURICH J F ET AL: "IDENTIFICATION OF DINSTINCT REGIONS OF 5' FLANKING DNA THAT MEDIATE CONSTITUTIVE, IFN-GAMMA, STAT1, AND TNF-BETA-REGULATED EXPRESSION OF THE CLASS II TRANSACTIVATOR GENE" JOURNAL OF IMMUNOLOGY, vol. 160, no. 1, janvier 1998 (1998-01), pages 233-240, XP002073465
- LENNON A -M ET AL: "ISOLATION OF A B-CELL-SPECIFIC PROMOTER FOR THE HUMAN CLASS II TRANSACTIVATOR" IMMUNOGENETICS, vol. 45, no. 4, 1997, pages 266-273, XP002051562 cité dans la demande
- PISKURICH J F ET AL: "Two distinct gamma interferon-inducible promoters of the major histocompatibility complex class II transactivator gene are differentially regulated by STAT1, interferon regulatory factor 1, and transforming growth factor beta" MOLECULAR AND CELLULAR BIOLOGY, (JAN 1999) VOL. 19, NO. 1, PP. 431-440., XP002108509

### LISTAGE DE SEQUENCE

<110> Transgene SA
<120> Méthode de criblage de composés capables d'inhiber la fixation entre le facteur de transcription STAT1 et le facteur de transcription USF1
<130> D17374
<140>
   <141>
<150> FR 98 02025
   <151> 1998-02-19
<160> 2
<170> PatentIn Vers. 2.0
<210> 1
   <211> 664
   <212> ADN
   <213> Homo sapiens
<400> 1
   ggggagaagt cagaggtaac cttgccccct ccctcaattc cagatgagga aattcaggcc 60
   tgaaaaggga aagtgaccac ctcaaagtct catgccttgg aggacccagc aggaatccaa 120
   gacctctgaa aaggaccggc agggctcttg ccacggctgg gggtgtggtc atggtaacac 180
   aggttttcca tccatggaag gtacctgagg gattttctct tcctccctag ggccagcatc 240
   agaggagtga atagctcagt tagctcatct caggggccat gtgccctcgg aggtggtttg 300
   ccactttcac ggttggactg agttggagag aaacagagac ccacccaggg gtggggacaa 360
   gctccctgca actcaggact tgcagatcac ttgcccaagt ggctccctag ctcctggctc 420
   ctggcccggg gcctgggact ctccccgaag tggggctggc cactgtgagg aaccgactgg 480
   aggcagggac ctcttggatg ccccaggcag ttgggatgcc acttctgata aagcacgtgg 540
   tggccacagt aggtgcttgg ttgctccaca gcctggcccg agctcagcgc tgcagaaaga 600
   aagtgaaagg gaaaaagaac tgcggggagg cggggaggta ggatgaccag cggacgagct 660
   gcca                                                              664
<210> 2
   <211> 4441
   <212> ADN
   <213> Homo sapiens
<400> 2
   cagacttgcc gcggccccag agctggcggg agggagaggc caccagcagc gcgcgcggga 60
   gcccggggaa cagcggcagc tcacagtgtg ccaccatgga gttggggccc ctagaaggtg 120
   gctacctgga gcttcttaac agcgatgctg accccctgtg cctctaccac ttctatgacc 180
   agatggacct ggctggagaa gaagagattg agctctactc agaacccgac acagacacca 240
   tcaactgcga ccagttcagc aggctgttgt gtgacatgga aggtgatgaa gagaccaggg 300
   aggcttatgc caatatcgcg gaactggacc agtatgtctt ccaggactcc cagctggagg 360
   gcctgagcaa ggacattttc aagcacatag gaccagatga agtgatcggt gagagtatgg 420
   agatgccagc agaagttggg cagaaaagtc agaaaagacc cttcccagag gagcttccgg 480
   cagacctgaa gcactggaag ccagctgagc cccccactgt ggtgactggc agtctcctag 540
   tgggaccagt gagcgactgc tccaccctgc cctgcctgcc actgcctgcg ctgttcaacc 600
   aggagccagc ctccggccag atgcgcctgg agaaaaccga ccagattccc atgcctttct 660
   ccagttcctc gttgagctgc ctgaatctcc ctgagggacc catccagttt gtccccacca 720
   tctccactct gccccatggg ctctggcaaa tctctgaggc tggaacaggg gtctccagta 780
   tattcatcta ccatggtgag gtgccccagg ccagccaagt accccctccc agtggattca 840
   ctgtccacgg cctcccaaca tctccagacc ggccaggctc caccagcccc ttcgctccat 900
   cagccactga cctgcccagc atgcctgaac ctgccctgac ctcccgagca aacatgacag 960
   agcacaagac gtcccccacc caatgcccgg cagctggaga ggtctccaac aagcttccaa 1020
   aatggcctga gccggtggag cagttctacc gctcactgca ggacacgtat ggtgccgagc 1080
   ccgcaggccc ggatggcatc ctagtggagg tggatctggt gcaggccagg ctggagagga 1140
   gcagcagcaa gagcctggag cgggaactgg ccaccccgga ctgggcagaa cggcagctgg 1200
   cccaaggagg cctggctgag gtgctgttgg ctgccaagga gcaccggcgg ccgcgtgaga 1260
   cacgagtgat tgctgtgctg ggcaaagctg gtcagggcaa gagctattgg gctggggcag 1320
   tgagccgggc ctgggcttgt ggccggcttc cccagtacga ctttgtcttc tctgtcccct 1380
   gccattgctt gaaccgtccg ggggatgcct atggcctgca ggatctgctc ttctccctgg 1440
   gcccacagcc actcgtggcg gccgatgagg ttttcagcca catcttgaag agacctgacc 1500
   gcgttctgct catcctagac gccttcgagg agctggaagc gcaagatggc ttcctgcaca 1560
   gcacgtgcgg accggcaccg gcggagccct gctccctccg ggggctgctg gccggccttt 1620
   tccagaagaa gctgctccga ggttgcaccc tcctcctcac agcccggccc cggggccgcc 1680
   tggtccagag cctgagcaag gccgacgccc tatttgagct gtccggcttc tccatggagc 1740
   aggcccaggc atacgtgatg cgctactttg agagctcagg gatgacagag caccaagaca 1800
   gagccctgac gctcctccgg gaccggccac ttcttctcag tcacagccac agccctactt 1860
   tgtgccgggc agtgtgccag ctctcagagg ccctgctgga gcttggggag gacgccaagc 1920
   tgccctccac gctcacggga ctctatgtcg gcctgctggg ccgtgcagcc ctcgacagcc 1980
   cccccggggc cctggcagag ctggccaagc tggcctggga gctgggccgc agacatcaaa 2040
   gtaccctaca ggaggaccag ttcccatccg cagacgtgag gacctgggcg atggccaaag 2100
   gcttagtcca acacccaccg cgggccgcag agtccgagct ggccttcccc agcttcctcc 2160
   tgcaatgctt cctgggggcc ctgtggctgg ctctgagtgg cgaaatcaag gacaaggagc 2220
   tcccgcagta cctagcattg accccaagga agaagaggcc ctatgacaac tggctggagg 2280
   gcgtgccacg ctttctggct gggctgatct tccagcctcc cgcccgctgc ctgggagccc 2340
   tactcgggcc atcggcggct gcctcggtgg acaggaagca gaaggtgctt gcgaggtacc 2400
   tgaagcggct gcagccgggg acactgcggg cgcggcagct gcttgagctg ctgcactgcg 2460
   cccacgaggc cgaggaggct ggaatttggc agcacgtggt acaggagctc cccggccgcc 2520
   tctcttttct gggcacccgc ctcacgcctc ctgatgcaca tgtactgggc aaggccttgg 2580
   aggcggcggg ccaagacttc tccctggacc tccgcagcac tggcatttgc ccctctggat 2640
   tggggagcct cgtgggactc agctgtgtca cccgtttcag ggctgccttg agcgacacgg 2700
   tggcgctgtg ggagtccctg cggcagcatg gggagaccaa gctacttcag gcagcagagg 2760
   agaagttcac catcgagcct ttcaaagcca agtccctgaa ggatgtggaa gacctgggaa 2820
   agcttgtgca gactcagagg acgagaagtt cctcggaaga cacagctggg gagctccctg 2880
   ctgttcggga cctaaagaaa ctggagtttg cgctgggccc tgtctcaggc ccccaggctt 2940
   tccccaaact ggtgcggatc ctcacggcct tttcctccct gcagcatctg gacctggatg 3000
   cgctgagtga gaacaagatc ggggacgagg gtgtctcgca gctctcagcc accttccccc 3060
   agctgaagtc cttggaaacc ctcaatctgt cccagaacaa catcactgac ctgggtgcct 3120
   acaaactcgc cgaggccctg ccttcgctcg ctgcatccct gctcaggcta agcttgtaca 3180
   ataactgcat ctgcgacgtg ggagccgaga gcttggctcg tgtgcttccg gacatggtgt 3240
   ccctccgggt gatggacgca agttcacggc tgccggggcc cagcagctcg ctgccagcct 3300
   tcggaggtgt cctcatgtgg agacgctggc gatgtggacg cccaccatcc cattcagtgt 3360
   ccaggaacac ctgcaacaac aggattcacg gatcagcctg agatgatccc agctgtgctc 3420
   tggacaggca tgttctctga ggacactaac cacgctggac cttgaactgg gtacttgtgg 3480
   acacagctct tctccaggct gtatcccatg aggcctcagc atcctggcac ccggcccctg 3540
   ctggttcagg gttggcccct gcccggctgc ggaatgaacc acatcttgct ctgctgacag 3600
   acacaggccc ggctccaggc tcctttagcg cccagttggg tggatgcctg gtggcagctg 3660
   cggtccaccc aggagccccg aggccttctc tgaaggacat tgcggacagc cacggccagg 3720
   ccagagggag tgacagaggc agccccattc tgcctgccca ggcccctgcc accctgggga 3780
   gaaagtactt cttttttttt atttttagac agagtctcac tgttgcccag gctggcgtgc 3840
   agtggtgcga tctgggttca ctgcaacctc cgcctcttgg gttcaagcga ttcttctgct 3900
   tcagcctccc gagtagctgg gactacaggc acccaccatc atgtctggct aatttttcat 3960
   ttttagtaga gacagggttt tgccatgttg gccaggctgg tctcaaactc ttgacctcag 4020
   gtgatccacc cacctcagcc tcccaaagtg ctggggatta caagcgtgag ccactgcacc 4080
   gggccacaga gaaagtactt ctccaccctg ctctccgacc agacaccttg acagggcaca 4140
   ccgggcactc agaagacact gatgggcaac ccccagcctg ctaattcccc agattgcaac 4200
   aggctgggct tcagtggcag gctgcttttg tctatgggac tcaatgcact gacattgttg 4260
   gccaaagcca aagctaggcc tggccagatg caccaggccc ttagcaggga aacagctaat 4320
   gggacactaa tggggcggtg agaggggaac agactggaag cacagcttca tttcctgtgt 4380
   cttttttcac tacattataa atgtctcttt aatgtcacaa aaaaaaaaaa aaaaaaaaaa 4440
   a 4441

## Description

La présente invention concerne une méthode permettant d'identifier des composés capables d'inhiber l'activation par les cytokines, notamment par l'interféron γ, de l'expression du gène CIITA qui lui même est impliqué dans le contrôle et la régulation de l'expression de gènes codant pour des molécules MHC de type II.

Les molécules du complexe majeur d'histocompatibilité (désigné ci-après MHC), de classe Il sont des glycoprotéines hétérodimériques transmembranaires directement impliquées dans l'activation des lymphocytes T helper CD4÷ au cours de la réponse immunitaire.

Chez l'homme, ce complexe de classe II est représenté par les molécules appartenant au système HLA (Human Leucocyte Antigen). Les gènes codant pour les chaînes α et β constituant les molécules HLA-DR, HLA-DQ et HLA-DP sont localisés au niveau de la région D du chromosome 6

L'expression de ces gènes est parfaitement régulée. Contrairement aux gènes codant pour les molécules MHC de type I qui sont exprimés de façon ubiquitaire, les gènes codant pour les molécules du MHC de classe II sont exprimés soit de manière constitutive uniquement dans quelques types cellulaires tels que les lymphocytes B, les lymphocytes T activés, les macrophages, les cellules de l'épithélium thymique, les cellules dendritiques telles que par exemple les cellules de Langerhans, soit de manière induite après stimulation, par exemple par des cytokines, et plus particulièrement par l'interféron γ (INF γ) ou l'interleukine 4 (IL4), dans plusieurs autres types cellulaires tels que par exemple les cellules appartenant à la lignée des macrophages ou des monocytes, les cellules endothéliales, les fibroblastes, les cellules musculaires ou les cellules cancéreuses telles que par exemple des cellules de mélanome.

En outre, dans les lymphocytes B, l'expression des gènes codant pour les molécules MHC de classe II, est transitoire. En effet, la différenciation des cellules B en plasmocytes produisant les immunoglobulines, s'accompagne de l' extinction de certains gènes, dont ceux codant pour le MHC de classe II.

De même, il a été montré que le taux d'expression des molécules MHC de type II constitue un facteur déterminant du processus d'activation des cellules T.

En conséquence, il apparaît clairement que les mécanismes moléculaires de régulation de l'expression de ces gènes constituent un élément clé de l'efficacité de la réponse immunitaire. Tout défaut dans ce procédé de régulation peut aboutir à d'importants troubles immunologiques, ou maladies auto-immunes. Ainsi, dans certains cas, une expression anormale des gènes MHC de classe II a été observée à la surface de cellules qui normalement ne devraient pas exprimer de tels gènes. De même, une sur-expression de ces gènes peut être observée qui conduit à une activation aberrante et non contrôlée des lymphocytes CD4+ [BOTTAZZO et al., 1986, Immunol. Rev., 94, 137-169]. De telles manifestations pourraient être, au moins en partie, responsables d'affections telles que le diabète insulino dépendant, la sclérose en plaque, la polyarthrite rhumatoïde ou le lupus érythémateux. A l'inverse, chez certains patients, une immunodéficience a pu être mise en évidence résultant d'un trouble au niveau de l'expression des gènes MHC de classe II. Citons pour exemple le syndrome BLS (bare lymphocytes syndrome) qui est une affection autosomique récessive pour laquelle l'expression des gènes MHC de classe II est très limitée, voire inexistante, ce qui se traduit par l'absence de réponses immunitaires cellulaire et humorale et s'accompagne de nombreuses infections, souvent fatales.

Plusieurs équipes scientifiques ont analysé les mécanismes de régulation de l'expression des gènes MHC de classe II et ont identifié un certain nombre de molécules transactivatrices susceptibles de se fixer, directement ou indirectement, sur des séquences promotrices spécifiques desdits gènes [pour une revue, voir MACH et al., 1996, Annu. Rev. Immunol. 14, 301-331].

Le déposant a précédemment identifié et caractérisé l'un de ces facteurs, le facteur CIITA (class II transactivator)[STEIMLE et al., 1993, Cell 75, 135-146 et EP 648836]. Le document WO 9606107 montre en outre qu'il existe deux domaines au sein du facteur CIITA plus impliqués dans l'activation de la transcription des gènes MHC de classe II. Néanmoins, de manière surprenante et contrairement à ce qui est observé pour les autres facteurs impliqués dans la régulation de l'expression des gènes MHC de classe II (COGSWELI, et al., 1991, Crit. Rev. Immunol. 11, 87-112), STEIMLE et al. ont montré que l'expression du facteur CIITA coïncide étroitement avec l'expression des gènes MHC de classe II et est absolument requise tant pour l'expression constitutive que pour l'induction desdits gènes MHC. Par ailleurs. SILACCI et al (1994, J. Exp. Med., 180, 1329-1336) ont montré que l'extinction des gènes MHC de classe Il lors de la différenciation des plasmocytes est associée à l'extinction du géne codant pour le facteur CIITA.

Par ailleurs, LENNON et al. (1997, Immunogenetics, 45, 266-273) ont identifié la séquence promotrice d'un gène CIITA responsable de l'expression différentielle de ce facteur dans les cellules B. Toutefois, l'existence de cette seule séquence ne permet pas d'expliquer pourquoi on observe une expression différentielle du facteur CIITA dans différents types cellulaires. En outre, elle ne rend pas compte de l'induction par les cytokines.

Dans des travaux antérieurs, le déposant a identifié, à partir d'échantillons issus de différents tissus d'origine humaine, l'organisation complexe des séquences assurant le contrôle de l'expression du facteur CIITA, isolé et caractérisé plusieurs régions promotrices et mis en évidence l'existence de différentes formes du facteur CIITA, et également de différents gènes CIITA. Ces travaux ont fait l'objet d'une publication (Muhlethaler-Mottet et al., 1997, EMBO J., 16, 2851-2860) et d'un dépôt d'une demande de brevet français n° 97/04954 (suivi d'un dépôt européen EP 0 847 049). Ainsi, les inventeurs ont montré que les différents promoteurs identifiés peuvent être activés de manière sélective : deux des promoteurs sont responsables de expression constitutive du gène CIITA dans les cellules dendritiques (promoteur I) et dans les lymphocytes B (promoteur III) alors que le promoteur IV intervient lors de l'expression du gène CIITA après induction par une cytokine, notamment l'interféron γ.

En outre, Piskurich et al. (1998, J. of immunobiology, 233-240) ont élucidé au sein du promoteur de CIITA les fragments responsables de l'expression de CIITA spécifique dans les cellules B, de l'effet suppressif de TGF-beta et de l'induction de l'expression par l'interféron gamma. Le rôle de régulation de STAT1 est également mis en lumière pour l'expression induite par l'interféron gamma.

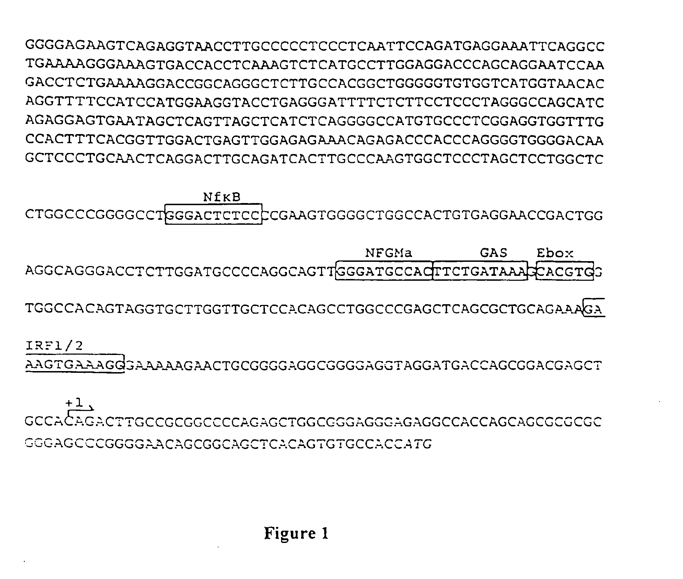

Plus particulièrement, les inventeurs ont identifié une séquence capable d'exprimer une activité de promoteur transcriptionnel après induction par une cytokine, telle que par exemple l'interféron γ ou l'interleukine 4. Une telle séquence est représentée par la séquence comprenant tout ou partie d'une séquence identifiée SEQ ID n°1, ou sa séquence complémentaire. L'analyse de cette séquence permet d'identifier plusieurs régions correspondant a des sites de régulation *en cis* de l'expression, tels que notamment le site NF-GMa, l'élément GAS, la boîte E ou le site de fixation du facteur IRF- (Muhlethaler-Mottet et al., 1997, EMBO J., 16. 2851-2860 et Figure 1).

Par ailleurs, le document WO95/28492 décrit des mutations dans la séquence de l'élément GAS permettant de trouver des agonistes et antagonistes de la voie de signalisation par l'interféron gamma et la protéine de régulation transcriptionnelle associée :STAT1.

Récemment, plusieurs travaux ont permis de mieux comprendre la succession d'événements et de signaux impliqués dans l'activation de gènes exprimés en réponse à l'induction par une cytokine, notamment par l'interféron γ. Un shéma d'activation a été proposé par Darnel et al. (1997, Science 277, 1630-1635). Selon ce modèle, dans un premier temps, la cytokine activatrice, l'interféron γ par exemple, se fixe sur ses récepteurs cellulaires de surface permettant ainsi l'activation des tyrosines kinases JAK1 et JAK2 cellulaires. Dans un second temps, les résidus tyrosine du facteur de transcription STAT1, localisé dans le cytoplasme des cellules, sont phosphorilés par les kinases JAK activées. Cette phosphorylation permet, dans un troisième temps, au facteur STAT1 activé de migrer dans le noyau où il se fixe sur la boite GAS des promoteurs inductibles par les cytokines (ex. Interféron γ) permettant ainsi l'expression activée des gènes placés sous le contrôle de tels promoteurs.

L'implication d'un td système d'activation JAK/STAT1 dans le contrôle de l'expression des gènes CIITA inductibles par l'interféron γ a fait l'objet d'études qui permettent de constater que, comme dans le cas d'autres gènes inductibles par l'interféron γ, l'expression du facteur CIIT A ne peut pas être induite dans des lignées cellulaires déficientes pour JAK1 (Chang et al., 1994, J. Exp Med., 180, 1367-1374).

De même, Meraz et al. (1996, Cell, 84, 431-442) ont montré que l'expression du gène CIITA n'est pas induite par l'interféron γ dans les macrophages de la moelle osseuse issue de souris STAT1, suggérant ainsi un rôle déterminant de STAT1 dans l'induction de l'expression du gène CIITA par l'interféron γ.

En outre, Lee et Benveniste (1996, J. Immunol., 157, 1559-1568) ont montré par des expériences utilisant des oligonucléotides antisens spécifiques de la séquence d'acide nucléique codant pour le facteur protéique STAT1, que la réduction de l'expression de la protéine STAT1 s'accompagne d'une réduction de l'expression du gène CIITA pouvant être observée après induction par l'interféron γ.

Enfin, il a été montré que le facteur STAT1 reconnaît spécifiquement une séquence d'acide nucléique particulière appelée «élément GAS» (Darnel et al. 1997, Science 277, 1630-1635). L'analyse de la séquence du promoteur IV du gène CIITA (inductible par les cytokines; Muhlethaler-Mottet et al., 1997, EMBO J., 16. 2851-2860 - et Figure 1) a révélé la présence d'une telle séquence.

Comme cela est précisé plus haut, le promoteur IV renferme également la séquence CACGTG (boîte E, Gregor et al., 1990, Genes Dev., 4, 1730-1740 , voir Figure 1). Ceci pourrait indiquer qu'un facteur de transcription appartenant à la famille des hélice/boucle/héilce/leucine zipper est susceptible d'intervenir dans la régulation de l'expression des gènes placés sous le contrôle du promoteur IV. Plusieurs acteurs appartenant à cette famille sont décrits dans la littérature, citons notamment les facteurs de transcription exprimés de manière constitutive tels que les facteurs TFE3 (Beckmann et al, 1990, Genes Dev., 4, 167-179), USF (Gregor et al., 1990, Genes Dev, 4, 1730-1740) et USF2 (Sirito et aL, 1994, Nucleic Acid Res. 22, 427-433) ou des protéines impliquées dans le système Myc telles que Myc-Max ou Mad-Max (Ayer et al., 1993, Cell 72, 211.222).

Plus particulièrement, le acteur de transcription USF 1 est exprimé de manière ubiquitaire et participe à la régulation de l'expression de différents gènes dont certains sont exprimés de manière «tissus spécifique» ou de façon inductible, tels que par exemple le gène codant pour l'hormone de croissance humaine (Peritz et al., 1988, J. Biol. Chem, 263, 5005-5007), le gène codant pour la chaîne λ2 des immunoglobulines (Chang et al., 1992, Nucleic Acid Res., 20, 287-293) ou encore le gène codant pour p53 (Reisman et Rotter, 1993, Nucleic Acid Res., 21, 345-350).

Par ailleurs, le document WO95/28482 décrit des séquences de nucléotides comprenant des éléments de régulation de l'ADN qui se lient, de manière directe ou indirecte, à des protéines de régulation transcriptionnelle telles que STAT1, après que ces dernières ont été activées par des cytokines.

Le document WO97/02354 quant à lui concerne l'identification d'un complexe comprenant le facteur de transcription DP-1 et le modulateur de transcription p53.

Aucun de ces documents ne mentionne le facteur de transcription USF1.

Le déposant a maintenant mis en évidence que les facteurs de transcription STAT1 et USF1 se fixent respectivement sur les élément GAS et sur la boîte E du promoteur IV. Toutefois, de manière très surprenante, le déposant a également montré que la fixation du facteur STAT1 sur le site GAS est fortement stabilisée par le facteur USF1 et que ces facteurs se lient de manière coopérative aux sites de fixation localisés sur le promoteur IV, cette interaction coopérative jouant un rôle déterminant dans le contrôle de l'activation spécifique du promoteur IV par les cytokines, en particulier par l'interféron γ.

Par l'expression «se lient de manière coopérative» on entend indiquer qu'il existe une interaction entre les facteurs protéiques dont il est question, qui peut avoir lieu avant ou après leur fixation sur leur site respectif, qui permet notamment de définir des sites d'interaction spécifiques entre lesdits facteurs protéiques et qui se traduit par un effet coopératif. Cet effet coopératif s'entend comme un résultat qui ne peut être observé que lorsque l'interaction dont il est question a lieu, par exemple cet effet coopératif consistera en une stabilisation de la fixation d'au moins un des facteurs protéiques sur son site (étant sous-entendu, grâce à l'interaction existant entre les facteurs protéiques). Selon un cas particulier, l'effet coopératif sera un effet synergique caractérisé en ce que l'effet observé par la mise en oeuvre des facteurs protéiques sera supérieur à l'effet attendu correspondant à la somme des effets individuels observés pour chaqu'un des facteurs.

Ce mécanisme d'induction de l'expression d'un gène placé sous le contrôle du promoteur IV se distingue des autres systèmes précédemment décrits pour les gènes inductibles par l'interféron γ en ce que celui-ci requiert le facteur USF1 en tant que partenaire essentiel pour la fixation et par conséquent pour l'activité du facteur STAT1. La découverte de ce mécanisme, impliqué en particulier dans l'activation par l'interféron γ de l'expression du gène CIITA, et par conséquent dans l'induction par l'interféron γ des molécules MHC de classe II, a conduit le déposant à mettre au point une nouvelle méthode d'identification de molécules capables d'inhiber l'expression de gènes placés sous le contrôle d'un promoteur dont l'activité est induite par la fixation coopérative de STAT1 1 et de USF1, et plus particulièrement de tout ou partie du promoteur IV. De manière préférée, ledit gène est le gène codant pour CIITA.

Par l'expression « séquence d'acide nucléique codant pour le polypeptide CIITA », on entend que la séquence en cause comprend tout ou partie d'une séquence d'acide nucléique correspondant aux ARNm issus de différents tissus ou lignées cellulaires exprimant une activité CIITA de manière constitutive ou après induction. Il peut donc s'agir aussi bien de séquences au moins partiellement codantes que par exemple de séquences intervenant dans le contrôle de l'expression, notamment des séquences ayant une activité de promoteur transcriptionnel.

Par «séquence d'acide nucléique», on entend un fragment d'ADN et/ou d'ARN, double brin ou simple brin, naturel isolé, ou de synthèse, désignant un enchaînement précis de nucléotides, modifiés ou non, permettant de définir un fragment ou une région d'un acide nucléique.

Par «polypeptide», on entend désigner un enchaînement précis d'aminoacides, indépendamment de sa taille ou de sa fonction, naturel isolé ou de synthèse, modifié ou non.

Par «séquence d'acide nucléique présentant une activité de promoteur transcriptionnel», on entend une séquence d'acide nucléique permettant de contrôler, c'est à dire d'initier et/ou de moduler, la transcription d'au moins un gène, homologue ou hétérologue, localisé en aval de ladite séquence. De même, on parlera de la fonction promotrice desdites séquences ou de promoteur.

Par «gène reporteur», on entend toute séquence d'acide nucléique localisée en aval d'une seconde séquence d'acide nucléique, permettant d'analyser l'activité de promoteur transcriptionnel de ladite seconde séquence. En effet, la transcription de ce gène reporteur se traduit par l'apparition d'un produit (ARN ou polypeptide) aisément détectable selon les techniques conventionnelles bien connues.

Par «facteur de transcription ou polypeptide STAT1» on entend désigner le facteur de transcription STAT1 qui est capable de se fixer au niveau de l'élément GAS des promoteurs inductibles pas l'interféron γ (Darnell, 1997, Science 277, 1630-1635).

Par «facteur de transcription ou polypeptide USF1 » on entend désigner le facteur de transcription USF1 qui est capable de se fixer au niveau de la boîte E de promoteurs tels que par exemple le promoteur du gène codant pour l'hormone de croissance humaine (Peritz et al., 1988, J. Biol. Chem., 263, 5005-5007, du gène codant pour la chaîne λ2 des immunoglobulines (Chang et al., 1992, Nucleic Acid Res., 20, 287-293) ou encore du gène codant pour p53 (Reisman et Rotter, 1993, Nucleic Acid Res., 21, 345-350).

Il convient en outre de préciser que lesdits facteurs STAT1 et USF1 peuvent être d'origine recombinante ou naturelle, et plus particulièrement consister en des facteurs disponibles dans des extraits cellulaires, en particulier nucléaires, préparés à partir de lignée cellulaire, éventuellement stimulée par une cytokine, en particulier par l'interféron γ, exprimant lesdits facteurs. En ce qui concerne plus particulièrement le facteur STAT1, celui ci peut être soit sous une forme non activée (non phosphorilée) soit sous une forme activée (phosphorilée, notamment par l'action de l'enzyme JAK1). Pour la réalisation de la présente invention on choisira de préférence la forme activée de STAT 1.

La présente invention concerne en premier lieu une méthode pour déterminer si un composé candidat est capable d'inhiber la fixation entre les polypeptides STAT1 et USF1 comprenant les étapes suivantes :
(a) disposer de tout ou partie du polypeptide STAT 1 présentant la propriété de se fixer au polypeptide USF1,
(b) disposer de tout ou partie du polypeptide USF 1 présentant la propriété de se fixer au polypeptide STAT1,
(c) mettre en contact lesdits polypeptides tels que définis en (a) et (b) avec undit composé candidat dans des conditions permettant la fixation entre les polypeptides STAT 1 et USF 1,
(d) mesurer la fixation entre les polypeptides STAT1 et USF1, et (e) comparer cette mesure avec la mesure de la fixation entre les polypeptides STAT 1 et USF1 observée dans les mêmes conditions expérimentales en absence dudit composé candidat, une diminution de la fixation permettant de conclure que ledit composé candidat est capable d'inhiber la fixation entre les polypeptides STAT1 et USF1.

Les polypeptides STAT 1 et USF1 utilisées dans le cadre de la présente invention peuvent soit être des polypeptides naturels, extraits par exemple de lignées cellulaires exprimant les gènes correspondants, telle que par exemple la lignée Me67.8, et plus particulièrement présents dans l'extrait nucléaire de telles lignées, lesdites lignées pouvant éventuellement être stimulées par l'interféron γ, soit des protéines recombinantes (Greenlund et al., 1995, Immunity, 2, 677-687 pour STAT 1 et Roy et al., 1991, Nature, 354, 245-248 pour USF1). La préparation d'extraits nucléaires à partir de cellules est une technique bien maitrisée par l'homme du métier. Les polypeptides STAT 1 et USF 1 mis en oeuvre dans la présente invention peuvent ou non conserver leur fonction activatrice d'autres gènes pour lesquels l'effet coopératif des deux polypeptides n'est pas observé (les polypeptides agissent alors séparément). Plus particulièrement il est possible d'utiliser une partie seulement desdits polypeptides STAT1 et/ou USF1 pour autant qu'ils aient conservé leurs propriétés de se fixer l'un à l'autre, et éventuellement la propriété de se fixer sur leurs sites respectifs. Selon un cas préféré de l'invention, le polypeptide STAT1 est capable de se fixer sur l'élément GAS (5'-TTCTGATAAA-3') parce qu'il est sous sa forme activée (phosphorilée). Cette phosphorilation du polypeptide STAT1 peut notamment être obtenues a) naturellement, dans une cellule exprimant ledit polypeptide induite par une cytokine, de préférence par l'interféron γ, b) par l'action d'une kinase, telle que par exemple JAK1 1 ou c) chimiquement, par synthèse.

Selon une première variante, l'étape (d) consiste en une mesure indirecte c'est à dire que dans ce cas particulier on détermine la formation de complexes comprenant STAT1. USF1 et une séquence d'acide nucléique double brin comportant l'élément GAS (5'-TTCTGATAA-3') et la boîte E (5'-CACGTG-3'). Ce type de méthode est largement décrit dans la littérature, repose généralement sur des expériences de migration électrophorétique (gel retard ou band shift en anglais) et est exemplifié dans la présente demande.

Selon une seconde variante, l'étape (d) consiste en une autre mesure indirecte c'est à dire que dans ce cas, on mesure l'expression d'une séquence d'acide nucléique codant pour tout ou partie d'un polypeptide, ladite expression étant placée sous le contrôle d'un promoteur renfermant au moins l'élément GAS (5'-TTCTGATAAA-3') et la boîte E (5'-CACGTG-3'), ou sa séquence complémentaire.

Selon un cas préféré de réalisation de l'invention, ladite séquence promotrice est sélectionnée parmi les séquences qui renferment tout ou partie du promoteur IV (SEQ ID N°1), ou de sa séquence complémentaire.

Selon l'invention, lesdites séquences d'acide nucléique dont l'expression est mesurée à l'étape (d) peuvent 1) être des gènes reporteurs tels que par exemple le gène de la β globine de lapin, de la luciférase ou de la β lactamase ou 2) coder pour tout ou partie de polypeptides ayant la séquence en acides aminés d'un facteur CIITA tel que décrit dans la demande de brevet français N° 97 04954 et plus particulièrement tel que défini par SEQ ID N°2. Selon ce dernier cas, on dira alors qu'elles codent pour tout ou partie du polypeptide CIITA

La mesure de l'expression de la séquence d'acide nucléique peut en particulier consister a) en la mesure des ARN messagers spécifiques exprimés à partir de ladite séquence d'acide nucléique ou b) en la mesure du polypeptide exprimé. Des exemples de telles méthodes sont largement développées dans la littérature et leur mise en oeuvre est à la portée de l'homme du métier. A titre d'exemples, on peut citer les techniques telles que celles reposant sur l'hybridation de sondes oligonucléiques marquées dont la séquence est spécifique des ARN que l'on cherche à détecter, l'amplification par exemple par PCR à l'aide d'amorces dont la séquence est spécifique desdits ARN, la technique de protection contre la dégradation par la RNAse, ... ou encore l'utilisation d'anticorps spécifiques de tout ou partie du polypeptide synthétisé, etc...

Selon un cas particulier et préféré de cette seconde variante de mise en oeuvre de l'invention, l'étape de mesure de l'expression de la séquence d'acide nucléique est réalisée dans des conditions permettant l'induction de ladite expression par une cytokine, et plus particulièrement par l'interféron γ.

Selon une troisième variante de l'invention, l'étape (d) consiste en une mesure directe de formation de complexes entre les polypeptides STAT1 et USF1, par exemple par utilisation d'anticorps spécifiques desdits complexes ou tout autre moyen approprié.

Les méthodes de mesure proposées dans les variantes exposées ci-dessus sont également adaptables pour les méthodes selon l'invention présentées ci-après.

L'invention concerne également une méthode pour déterminer si un composé candidat est capable d'inhiber l'expression d'une séquence d'acide nucléique codant pour tout ou partie d'un polypeptide, de préférence pour tout ou partie du polypeptide CIITA (SEQ ID N°2) ou pour tout ou partie d'un gène reporteur placée sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N°1), ou leur séquence complémentaire respective comprenant les étapes suivantes :
(a) disposer de tout ou partie d'un polypeptide STAT1 présentant la propriété de se fixer à la protéine USF1 et sur l'élément GAS (5'-TTCTGATAAA-3'),
(b) disposer de tout ou partie de la protéine USF1 présentant la propriété de se fixer à la protéine STAT1 et sur la boîte E (5'-CACGTG-3'),
(c) disposer d'une séquence d'acide nucléique codant pour tout ou partie d'un polypeptide , de préférence pour tout ou partie du polypeptide CIITA (SEQ ID N°2) ou pour tout ou partie d'un gène reporteur, dont l'expression est placée sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N°1),
(d) mettre en contact lesdits polypeptides tels que définis en (a) et (b), une dite séquence d'acide nucléique telle que définie en (c) et undit composé candidat,
(e) mesurer l'expression de ladite séquence d'acide nucléique, et (f) comparer cette mesure avec la mesure de l'expression de ladite séquence d'acide nucléique observée dans les mêmes conditions expérimentales, notamment d'activation de l'expression, en absence dudit composé candidat, une diminution de ladite expression permettant de conclure que ledit composé candidat est capable d'inhiber l'expression d'une séquence d'acide nucléique codant pour tout ou partie d'un polypeptide, notamment pour tout ou partie du polypeptide CIITA ou pour tout
ou partie d'un gène reporteur placé sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N°1).

L'invention concerne également une méthode pour déterminer si un composé candidat est capable d'inhiber l'activation par une cytokine, plus particulièrement par l'interféron γ, de l'expression d'une séquence d'acide nucléique codant pour tout ou partie d'un polypeptide, notamment pour tout ou partie du polypeptide CIITA (SEQ ID N°2) ou pour tout ou partie d'un gène reporteur, placé sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N°1), comprenant les étapes suivantes:
(a) disposer d'une lignée cellulaire exprimant les polypeptides STAT1 et USF1 naturels et fonctionnels, par exemple la lignée Me67.8,
(b) transfecter ladite lignée cellulaire avec un vecteur d'expression comprenant au moins une séquence d'acide nucléique codant pour tout ou partie d'un polypeptide, notamment pour tout ou partie du polypeptide CIITA (SEQ ID N°2) ou pour tout ou partie d'un gène reporteur, place sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N° 1).
(c) mettre lesdites cellules en contact avec undit composé candidat ou transfecter les cellules avec un vecteur d'expression permettant l'expression d'undit composé à l'intérieur desdites cellules, dans des conditions autorisant l'activation de l'expression de la séquence d'acide nucléique par une cytokine, de préférence par l'interféron γ
(d) mesurer l'expression de ladite une séquence d'acide nucléique,
et (e) comparer cette mesure avec la mesure de l'expression de ladite séquence d'acide nucléique observée dans les mêmes conditions expérimentales, notamment d'activation de l'expression, en absence dudit composé candidat, une diminution de ladite expression permettant de conclure que ledit composé candidat est capable d'inhiber l'activation par une cytokine, plus particulièrement par l'interféron γ, de l'expression d'une séquence d'acide nucléique codant pour tout ou partie d'un polypeptide, notamment pour tout ou partie du polypeptide CIITA (SEQ ID N°2) ou pour tout ou partie d'un gène reporteur, placé sous le contrôle de tout ou partie d'un promoteur renfermant au moins l'élément GAS (5'-TTCTGATAAA-3') et la boîte E (5'-CACGTG-3'), et de manière préférée d'un promoteur IV (SEQ ID N°1).

Un vecteur d'expression comprenant au moins une séquence d'acide nucléique codant pour tout ou partie d'un polypeptide placé sous le contrôle de tout
ou partie du promoteur IV renfermant l'élément GAS (5'-TTCTGATAAA-3') et la boîte E (5'-CACGTG-3') peut notamment consister en un vecteur tel que décrit dans la demande de brevet français N° 97 04954.

L'invention concerne également des méthodes telles que définies ci dessus permettant d'identifier des composés candidat susceptibles d'inhiber l'expression des gènes codant pour les molécules MHC de classe II lorsque celle-ci est désirée, notamment dans des conditions pour lesquelles on souhaite agir après induction par une cytokine, et plus particulièrement par l'interféron γ.

De nombreuses affections, directement ou indirectement, liées à un trouble de l'expression des gènes codant pour les molécules MHC de classe II sont décrites dans la littérature. Citons pour exemple, des affections telles que le diabète insulino dépendant, la sclérose en plaque, la polyarthrite rhumatoïde ou le lupus érythémateux dont l'une des composantes pourrait être une sur-expression des gènes codant pour les molécules MHC de classeII.

D'autres caractéristiques et avantages de la présente invention apparaîtront à la lecture des exemples ci-après, illustrés par la figure 1. Toutefois, l'invention ne saurait se limiter au contenu desdits exemples.

La Figure 1 présente les différents éléments impliqués dans la régulation de l'expression induite par les cytokines des gènes placés sous le contrôle du promoteur IV.

### EXEMPLES.

### Matériel et Méthodes

### Les cellules

Les lignées cellulaires Me67.8 (mélanome) et THP1 (monocyte) sont cultivées dans un milieu RPMI-1640. Les lignées 2FTGH (fibrosarcome) et U3A (lignée 2FTGH n'exprimant pas STAT1) sont cultivées sur milieu de Dulbecco modifié. Les milieux sont complémentés avec 10% de sérum de veau faetal, 10 U/ml de pénicilline, 10 mg/ml de streptomycine et 2 mM de glutamine. Les incubation sont réalisées à 37°C sous 5% de CO2.

### Gènes reporteurs.

L'expression des gènes reporteur est mesurée par RT-PCR quantitative comme décrit dans Sperisen et al., 1992, PCR Meth Appli. 1, 164-170. Les transfections, la préparation des ARN et les analyses par RT-PCR sont réalisées comme précédemment décrit (Muhlethaler-Mottet et al., 1997, EMBO J., 16, 2851-2860). Le plasmide PIV-308 renfermen le fragment -308 à +75 de la région flanquante du promoteur IV du gène CIITA sous cloné en aval du gène codant pour la béta globuline de lapin du plasmide pGβG(+). L'activité du promoteur est mesurée par Phosphoimager.

### Tests de protection de la dégradation par la RNAse.

Les essais sont réalisés sur 10 µg d'ARN par réaction selon la technique décrite dans Steimle et al., 1993, Cell, 75, 135-146.

### Oligonucléotides utilisés.

### Analyse de la mobilité électrophorétique (EMSA)

Les cellules mises en oeuvre sont ou non stimulées par l'interféron γ (500U/ml) pendant 30 min avant la préparation des extraits nucléaires selon la méthode décrite par Harroch et al., 1994, EMBO J., 13, 1942-1949.

Les oligonucléotides radiomarqués à l'une de leur extrémités par l'addition de [γ-32P] ATPsont hybridés à leur séquence complémentaire et purifiés par électrophorèse sur gel de polyacrylamide de manière à obtenir des sondes marquées d'ADN double brin correspondant à tout ou partie du promoteur IV de CIITA.

Pour l'étude de fixation des facteurs protéiques sur la sonde NGE, 6µg de protéines extraites des noyaux des cellules sont mélangés avec 2.10⁴ cpm de sonde d'ADN, 1.25 µg de poly (dI) (dC) (Pharmacia), 0.5 µg d'ADN simple brin de E. Coli, avec ou sans compétiteur, dans un volume final de 20 µl [20 mM Tris-HCl pH 7.9, 50 mM NaCl, 1 mM EDTA, 5% (v/v) glycérol, mM dithiothréitol, 1 mM spermine et 100 µg de BSA]. Les conditions de fixation des facteurs sur la sonde G sont identiques hormis pour : pas de poly (dI) (dC), pas d'ADN de E coli.

La protéine STAT1 recombinante, putifiée et activée et la protéine USF1 purifiée et recombinante sont utilisées dans des conditions identiques.

Après addition de la sonde nucléique radiomarquée, le mélange est incubé pendant 30 minutes à 20°C.

Pour les expériences de «supershift», les anticorps sont ajoutés et le mélange est placé 20 minutes à 4°C avant de réaliser une électrophorèse sur gel de polyacrylamide 5%. Après séqhage, le gel est autoradiographié. Les résultats sont déterminés par PhosphoImager.

### Exemple 1: Mise en évidence de deux types de complexe pouvant se former au niveau de la région GAS/boite E.

Afin d'étudier les complexes ADN/Protéine qui peuvent se former au niveau de la région renfermant les éléments de régulation en cis GAS/boite E du promoteur IV inductible par l'interféron γ que le déposant avait précédemment identifié (Muhlethaler-Mottet et al., 1997, EMBO J., 16, 2851-2860), des essais de mobilité électrophorétiques ont été réalisés avec des sondes ADN couvrant cette région (NGE) et avec l'extrait nucléaire des cellules Me67.8, après ou sans induction par l'interféron γ.

Avec l'extrait nucléaire préparé à partir de cellules non stimulées, on constate qu'un complexe, noté L pour «lower», est formé entre l'ADN sonde et au moins une protéine présente dans l'extrait nucléaire.

Avec l'extrait nucléaire préparé à partir de cellules stimulées par l'interféron γ, on constate qu'un autre complexe est formé, noté U pour «upper» qui présente une mobilité électrophorétique moindre.

Afin d'étudier la spécificité et le site de fixation à l'ADN de chacun des complexes identifiés, nous avons réalisé des expériences de compétition en présence de sondes spécifiques de l'élement GAS (G) et de la boîte E (E). Les résultats montrent que le complexe L est seulement déstabilisé en présence de la sonde de compétition E, que l'extrait nucléaire provienne de cellules stimulées ou non par l'interféron γ. Par contre, la formation du complexe U est inhibée par les sondes de compétion G et E. Ces résultats indiquent par conséquent que le complexe L fait intervenir une protéine capable de se fixer sur la boîte E, qui est présente dans les cellules non stimulées et qu'après stimulation par l'interféron γ, un nouveau complexe peut se former. Nous avons par ailleurs montré par des expériences de mutagénèse dirigées sur les élements GAS et boîte E que la fixation de ce complexe U sur l'ADN n'est possible qu'en présence des séquences GAS et boîte E sauvages.

Ces expériences permettent de conclure que le complexe U est formé d'au moins un facteur capable de se lier à la boîte E et d'une protéine activée par l'interféron γ qui se lie à l'élément GAS. Un candidat possible pour ce second facteur protéique est la protéine STAT1 (Decker et al., 1991, EMBO J., 10, 927-932).

### Exemple 2 :

Afin d'évaluer directement le rôle de STAT1 dans la régulation de l'expression du gène CIITA après activation par l'interféron γ, la capacité d'induction du gène CIITA a été étudiée dans une lignée cellulaire déficiente pour STAT1 (U3A) et dans une lignée cellulaire exprimant STAT1 (2FTGH), dans des conditions d'activation par l'interféron γ.

Contrairement à ce que l'on observe dans la lignée 2FTGH, dans la lignée U3A, l'expression des ARN messagers de CIITA ou l'activation du promoteur IV de CIITA ne sont pas induits par la cytokine comme le montrent des expériences de protection à la RNAse et l'analyse de la fonction promotrice par construction de gène reporteurs exprimés sous le contrôle du promoteur à analyser.

Les résultats indiquent que STAT1 contrôle l'activation par l'interféron γ du promoteur IV de CIITA. Ces résultats sont en accord avec les travaux de Merraz et al., 1996, Cell, 84, 431-442 montrant que les ARN messagers de CIITA ne peuvent pas être induits par l'interféron γ dans les macrophages issus de souris STAT -/-.

Afin d'analyser le rôle de STAT1 au niveau du promoteur IV de CIITA et sa présence au niveau du complexe U, ledit complexe protéique, associé à la sonde NGE marquée a été analysé par «supershift» avec des anticorps monoclonaux spécifiques de STAT1. Alors que des anticorps monoclonaux non spécifiques n'ont aucun effet sur la migration éléctrophorétique des complexes ADN/protéine U, les anticorps monoclonaux anti-STAT 1 retardent considérablement la migration du complexe U, même pour de fortes dilutions (1/2000), alors que la migration du complexe L demeure inchangée. Ceci confirme que la protéine STAT1 est bien l'un des composants du complexe U.

### Exemple 3 :

Des essais fonctionnels ont permis de mettre en évidence le rôle majeur joué par la boîte E lors de l'induction par l'interféron γ. La séquence nucléique de la boîte E du promoteur IV de CIITA présente la séquence consensus CACGTG précédemment décrite comme site de fixation sur l'ADN des protéines hélice/boucle/hélice/leucine zipper.

L'utilisation d'anticorps spécifiques du facteur USF1 ont permis de montrer la présence de ce facteur au sein des complexes protéiques U et L. Afin de confirmer que le facteur USF1 est capable de se fixer sur le promoteur IV de CIITA, des expériences de EMSA ont été réalisées en présence de protéine recombinantes USF1. Les résultats montrent que la protéine USF1 est effectivement présente dans les complexes U et L et se fixe spécifiquement sur le promoteur IV de CIITA.

### Exemple 4 :

Nous avons ensuite analysé si les protéines STAT1 et USF1 formaient le complexe U de manière coopérative après induction par l'interféron γ. Pour cela, des analyses EMSA ont permis de montrer que contrairement au facteur recombinant USF1 qui peut, seul, se fixer sur la sonde radiomarquée NGE, le facteur STAT1 ne peut pas, par lui-même, se fixer sur la même sonde.Par ailleurs, l'addition de quantités croissantes de STAT1 activé à une quantité donnée d'USF 1, en présence d'un excès de sonde NGE libre, favorise la formation de complexes STAT1/USF1 plutôt que la fixation de l'USF1 seul. La quantité de sonde associée aux deux facteurs montre que la fixation de STAT1 et de USF1 est effectuée de manière coopérative car les quantités de sonde liée audit complexe (17%) est environ deux fois plus élevée que la somme de sonde fixée au facteur USF1 seul (7.6%) et au facteur STAT 1 seul (0%).

Cette fixation coopérative est également observée avec des extraits nucléaires issus de cellules non stimulées Me67.8 et de facteur STAT1 recombinant activé. Les complexes formés avec les protéines recombinantes présentent les mêmes profils de migration électrophorétiques que ceux obtenus avec des extraits nucléaires de cellules Me67.8 stimulées par l'interféron γ.

## Revendications

1. Méthode pour déterminer si un composé candidat est capable d'inhiber la fixation entre les polypeptides STAT1 et USF1 comprenant les étapes suivantes :
(a) disposer de tout ou partie du polypeptide STAT1 présentant la propriété de se fixer au polypeptide USF1,
(b) disposer de tout ou partie du polypeptide USF1 présentant la propriété de se fixer au polypeptide STAT1,
(c) mettre en contact lesdits polypeptides tels que définis en (a) et (b) avec undit composé candidat dans des conditions permettant la fixation entre les polypeptides STAT1 et USF1,
(d) mesurer la fixation entre les polypeptides STAT1 et USF1,
et (e) comparer cette mesure avec la mesure de la fixation entre les polypeptides STAT1 et USF1 observée dans les mêmes conditions expérimentales en absence dudit composé candidat, une diminution de la fixation permettant de conclure que ledit composé candidat est capable d'inhiber la fixation entre les polypeptides STAT1 et USF1.

2. Méthode selon la revendication 1 **caractérisée en ce que** l'étape (d) consiste en la mesure de la formation de complexes comprenant STAT1, USF1 et une séquence d'acide nucléique double brin comportant l'élément GAS :5'-TTCTGATAAA-3' et la boîte E :5'-CACGTG-3'.

3. Méthode selon la revendication 2 **caractérisée en ce que** ladite séquence d'acide nucléique double brin renferme tout ou partie du promoteur IV, de séquence SEQ ID N°1, ou de sa séquence complémentaire.

4. Méthode pour déterminer si un composé candidat est capable d'inhiber l'expression d'une séquence d'acide nucléique codant pour tout ou partie d'un polypeptide placé sous le contrôle de tout ou partie d'un promoteur renfermant l'élément GAS :5'-TTCTGATAAA-3' et la boîte E :5'-CACGTG-3' comprenant les étapes suivantes:
(a) disposer de tout ou partie d'un polypeptide STAT1 présentant la propriété de se fixer à la protéine USF1 et sur l'élément GAS :5'-TTCTGATAAA-3',
(b) disposer de tout ou partie d'un polypeptide USF1 présentant la propriété de se fixer à la protéine STAT1 et sur la boîte E :5'-CACGTG-3',
(c) disposer d'une séquence d'acide nucléique codant pour tout ou partie d'un polypeptide dont l'expression est placée sous le contrôle de tout ou partie d'un promoteur renfermant l'élément GAS :5'-TTCTGATAAA-3' et la boîte E :5'-CACGTG-3',
(d) mettre en contact lesdits polypeptides tels que définis en (a) et (b), une dite séquence d'acide nucléique telle que définie en (c) et undit composé candidat,
(e) mesurer l'expression de ladite une séquence d'acide nucléique,
et (f) comparer cette mesure avec la mesure de l'expression de ladite séquence d'acide nucléique observée dans les mêmes conditions expérimentales, notamment d'activation de l'expression, en absence dudit composé candidat, une diminution de ladite expression permettant de conclure que ledit composé candidat est capable d'inhiber l'expression d'une séquence d'acide nucléique codant pour tout ou partie d'un polypeptide placé sous le contrôle de tout ou partie d'un promoteur renfermant l'élément GAS :5'-TTCTGATAAA-3' et la boîte E :5'-CACGTG-3'.

5. Méthode pour déterminer si un composé candidat est capable d'inhiber l'activation par une cytokine de l'expression d'une séquence d'acide nucléique codant pour tout ou partie d'un polypeptide placé sous le contrôle de tout ou partie d'un promoteur renfermant l'élément GAS :5'-TTCTGATAAA-3' et la boîte E :5'-CACGTG-3' comprenant les étapes suivantes:
(a) disposer d'une lignée cellulaire exprimant les polypeptides STAT1 et USF1 naturels et fonctionnels,
(b) transfecter ladite lignée cellulaire avec un vecteur d'expression comprenant au moins une séquence d'acide nucléique codant pour tout ou partie d'un polypeptide placé sous le contrôle de tout ou partie d'un promoteur renfermant l'élément GAS :5'-TTCTGATAAA-3' et la boîte E :5'-CACGTG-3',
(c) mettre lesdites cellules en contact avec undit composé candidat ou transfecter les cellules avec un vecteur d'expression permettant l'expression d'undit composé à l'intérieur desdites cellules, dans des conditions autorisant l'activation de l'expression de la séquence d'acide nucléique par une cytokine,
(d) mesurer l'expression de ladite une séquence d'acide nucléique,
et (e) comparer cette mesure avec la mesure de l'expression de ladite une séquence d'acide nucléique observée dans les mêmes conditions expérimentales, notamment d'activation de l'expression, en absence dudit composé candidat, une diminution de ladite expression permettant de conclure que ledit composé candidat est capable d'inhiber l'activation par une cytokine de l'expression d'une séquence d'acide nucléique codant pour tout ou partie d'un polypeptide placé sous le contrôle de tout ou partie d'un promoteur renfermant l'élément GAS :5'-TTCTGATAAA-3' et la boîte E:5'-CACGTG-3'.

6. Méthode selon la revendication 5 **caractérisée en ce que** la cytokine est l'interféron γ.

7. Méthode selon la revendication 5 **caractérisée en ce que** la lignée cellulaire est la lignée Me67.8.

8. Méthode selon les revendications 4 à 7 **caractérisée en ce que** ladite séquence d'acide nucléique code pour tout ou partie du polypeptide CIITA de séquence SEQ ID N°2.

9. Méthode selon les revendications 4 à 7 **caractérisée en ce que** ladite séquence d'acide nucléique code pour tout ou partie d'un gène reporteur, plus particulièrement pour tout ou partie de la β globine de lapin , de la luciférase ou de la β lactamase.

10. Méthode selon les revendications 4 à 7 **caractérisée en ce que** ledit promoteur renfermant l'élément GAS :5'-TTCTGATAAA-3' et la boîte E :5'-CACGTG-3'est le promoteur IV de séquence SEQ ID N°1.

11. Méthode selon les revendications 4 à 10 **caractérisée en ce que** la mesure de l'expression de ladite séquence d'acide nucléique consiste en la mesure des ARN messagers spécifiques exprimés à partir de ladite séquence d'acide nucléique.

12. Méthode selon les revendications 4 à 10 **caractérisée en ce que** la mesure de l'expression de ladite séquence d'acide nucléique consiste en la mesure du polypeptide exprimé.

## Claims

1. A method for determining whether a candidate compound is capable of inhibiting binding between STAT1 and USF1 polypeptides, comprising the following steps:
(a) providing all or a portion of the STAT1 polypeptide having the property of binding to the USF1 polypeptide;
(b) providing all or a portion of the USF1 polypeptide having the property of binding to the STAT1 polypeptide;
(c) bringing said polypeptides as defined in (a) and (b) into contact with said candidate compound under conditions which enable binding between the STAT1 and USF1 polypeptides;
(d) measuring the binding between the STAT1 and USF1 polypeptides; and
(e) comparing this measurement with that for binding between the STAT1 and USF1 polypeptides observed under the same experimental conditions in the absence of said candidate compound, a reduction in binding allowing the conclusion that said candidate compound is capable of inhibiting binding between the STAT1 and USF1 polypeptides.

2. A method according to claim 1, **characterized in that** step (d) consists of measuring the formation of complexes comprising STAT1, USF1 and a double stranded nucleic acid sequence comprising the GAS element: 5'-TTCTGATAAA-3' and the E-box: 5'-CACGTG-3'.

3. A method according to claim 1, **characterized in that** said double stranded nucleic acid sequence comprises all or a portion of the promoter IV, sequence SEQ ID N°1, or of its complementary sequence.

4. A method for determining whether a candidate compound is capable of inhibiting expression of a nucleic acid sequence coding for all or a portion of a polypeptide placed under the control of all or a portion of a promoter comprising the GAS element: 5'-TTCTGATAAA-3' and the E-box: 5'-CACGTG-3', comprising the following steps:
(a) providing all or a portion of the STAT1 polypeptide having the property of binding to the USF1 protein and on the GAS element: 5'-TTCTGATAAA-3';
(b) providing all or a portion of the USF1 polypeptide having the property of binding to the STAT1 protein and on the E-box: 5'-CACGTG-3';
(c) providing a nucleic acid sequence coding for all or a portion of a polypeptide the expression of which is placed under the control of all or a portion of a promoter comprising the GAS element: 5'-TTCTGATAAA-3' and the E-box: 5'-CACGTG-3';
(d) bringing said polypeptides as defined in a) and b), said nucleic acid sequence as defined in c) and said candidate compound into contact;
(e) measuring the expression of said nucleic acid sequence; and
(f) comparing this measurement with that of the expression of said nucleic acid sequence observed under the same experimental conditions, in particular of activation of expression, in the absence of said candidate compound, a reduction in said expression allowing the conclusion that said candidate compound is capable of inhibiting expression of a nucleic acid sequence coding for all or a portion of a polypeptide placed under the control of all or a portion of a promoter comprising the GAS element: 5'-TTCTGATAAA-3' and the E-box 5' -CACGTG-3' .

5. A method for determining whether a candidate compound is capable of inhibiting activation by a cytokine of the expression of a nucleic acid sequence coding for all or a portion of a polypeptide placed under the control of all or a portion of a promoter comprising the GAS element: 5'-TTCTGATAAA-3' and the E-box 5'-CACGTG-3', comprising the following steps:
(a) providing a cell line expressing natural and functional STAT1 and USF1 polypeptides;
(b) transfecting said cell line with an expression vector comprising at least one nucleic acid sequence coding for all or a portion of a polypeptide placed under the control of all or a portion of a promoter comprising the GAS element: 5'-TTCTGATAAA-3' and the E-box 5'-CACGTG-3';
(c) bringing said cells into contact with a said candidate compound or transfecting the cells with an expression vector enabling expression of a said compound inside said cells, under conditions enabling activation of expression of the nucleic acid sequence by a cytokine;
(d) measuring the expression of said nucleic acid sequence; and
(e) comparing this measurement with that of the expression of said nucleic acid sequence observed under the same experimental conditions, in particular of activation of expression, in the absence of said candidate compound, a reduction in said expression allowing the conclusion that said candidate compound is capable of inhibiting activation by a cytokine of the expression of a nucleic acid sequence coding for all or a portion of a polypeptide placed under the control of all or a portion of a promoter comprising the GAS element: 5'-TTCTGATAAA-3' and E-box 5'-CACGTG-3'.

6. A method according to claim 5, **characterized in that** said cytokine is γ interferon.

7. A method according to claim 5, **characterized in that** the cell line is the Me67.8 line.

8. A method according to claims 4 to 7, **characterized in that** said nucleic acid sequence codes for all or a portion of the CIITA polypeptide (SEQ ID N° 2).

9. A method according to claims 4 to 7, **characterized in that** said nucleic acid sequence codes for all or a portion of a reporter gene, more particularly for all or a portion of rabbit β globin, luciferase or β lactamase.

10. A method according to claims 4 to 7, **characterized in that** said promoter comprising the GAS element: 5'-TTCTGATAAA-3' and the E-box 5'-CACGTG-3' is promoter IV (SEQ ID N° 1).

11. A method according to claims 4 to 10, **characterized in that** expression of said nucleic acid sequence is measured by measuring the specific messenger RNA expressed from said nucleic acid sequence.

12. A method according to claims 4 to 10, **characterized in that** expression of said nucleic acid sequence is measured by measuring the polypeptide expressed.

## Patentansprüche

1. Verfahren zur Bestimmung, ob eine Kandidatverbindung befähigt ist, die Bindung der Polypeptide STAT1 und USF1 zu inhibieren, welches die folgenden Stufen umfasst:
(a) Vorlegen von allem oder einem Teil des Polypeptids STAT1, das die Eigenschaft aufweist, am Polypeptid USF1 gebunden zu werden,
(b) Vorlegen von allem oder einem Teil des Polypeptids USF1, das die Eigenschaft aufweist, am Polypeptid STAT1 gebunden zu werden,
(c) Inkontaktbringen der in (a) und (b) definierten genannten Polypeptide mit einer genannten Kandidatverbindung unter Bedingungen, die die Bindung zwischen den Polypeptiden STAT1 und USF1 ermöglichen,
(d) Messen der Bindung zwischen den Polypeptiden STAT1 und USF1 und
(e) Vergleichen dieser Messung mit der Messung der Bindung zwischen den Polypeptiden STAT1 und USF1, die unter den gleichen Versuchsbedingungen in Abwesenheit der genannten Kandidatverbindung zu beobachten ist, wobei die Verringerung der Bindung Rückschlüsse ermöglicht, ob die genannte Kandidatverbindung befähigt ist, die Bindung zwischen den Polypeptiden STAT1 und USF1 zu inhibieren.

2. Verfahren gemäß Anspruch 1, **dadurch gekennzeichnet, dass** die Stufe (d) auf der Messung der Bindung von Komplexen beruht, die STAT1, USF1 und eine doppelsträngige Nucleinsäuresequenz umfasst, die das Element GAS: 5'-TTCTGATAAA-3' und die Box E: 5'-CACGTG-3' umfasst.

3. Verfahren gemäß Anspruch 2, **dadurch gekennzeichnet, dass** die genannte doppelsträngige Nucleinsäuresequenz alles oder einen Teil des Promotors IV, der Sequenz SEQ ID NO: 1, oder ihrer komplementären Sequenz umfasst.

4. Verfahren zur Bestimmung der Befähigung einer Kandidatverbindung zur Inhibierung der Exprimierung einer Nucleinsäuresequenz, die für das ganze oder einen Teil eines Polypeptids unter der Steuerung von allem oder einem Teil eines Promotors codiert, der das Element GAS: 5'-TTCTGATAAA-3' und die Box E: 5'-CACGTG-3' umfasst, wobei das Verfahren die folgenden Stufen umfasst:
(a) Vorlegen von allem oder einem Teil eines Polypeptids STAT1, das die Eigenschaft aufweist, an das Protein USF1 und auf dem Element GAS: 5'-TTCTGATAAA-3' gebunden zu werden,
(b) Vorlegen von allem oder einem Teil eines Polypeptids USF1, das die Eigenschaft aufweist, an das Protein STAT1 und auf der Box E: 5'-CACGTG-3' gebunden zu werden,
(c) Vorlegen einer Nucleinsäuresequenz, die für alles oder einen Teil eines Polypeptids codiert, das unter der Steuerung von allem oder einem Teil eines Promotors exprimiert wird, der das Element GAS: 5'-TTCTGATAAA-3' und die Box E: 5'-CACGTG-3' umfasst,
(d) Inkontaktbringen der in (a) und (b) definierten genannten Polypeptide, einer in (c) definierten genannten Nucleinsäuresequenz und einer genannten Kandidatverbindung,
(e) Messen der Exprimierung der genannten Nucleinsäuresequenz und
(f) Vergleichen dieser Messung mit der Messung der Exprimierung der genannten Nucleinsäuresequenz, die unter den gleichen Versuchsbedingungen, insbesondere der Aktivierung der Exprimierung, in Abwesenheit der genannten Kandidatverbindung beobachtet wird, wobei eine Verringerung der genannten Exprimierung Rückschlüsse auf die Befähigung der genannten Kandidatverbindung zur Inhibierung der Exprimierung einer Nucleinsäuresequenz ermöglicht, die für alles oder einen Teil eines Polypeptids unter der Steuerung von allem oder einem Teil eines Promotors codiert, der das Element GAS: 5'-TTCTGATAAA-3' und die Box E: 5'-CACGTG-3' umfasst.

5. Verfahren zur Bestimmung der Befähigung einer Kandidatverbindung zur Inhibierung der durch ein Zytokin bewirkten Aktivierung der Exprimierung einer Nucleinsäuresequenz, die für alles oder einen Teil eines Polypeptids unter Steuerung von allem oder einem Teil eines Promotors codiert, der das Element GAS: 5'-TTCTGATAAA-3' und die Box E: 5'-CACGTG-3' umfasst, wobei das Verfahren die folgenden Stufen umfasst:
(a) Vorlegen einer Zelllinie, die die natürlichen und funktionellen Polypeptide STAT1 und USF1 exprimiert,
(b) Transfizieren der genannten Zelllinie mit einem Expressionsvektor, der mindestens eine Nucleinsäuresequenz umfasst, die für alles oder einen Teil eines Polypeptids unter der Steuerung von allem oder einem Teil eines Promotors codiert, der das Element GAS: 5'-TTCTGATAAA-3' und die Box E: 5'-CACGTG-3' umfasst,
(c) Inkontaktbringen der genannten Zellen mit einer Kandidatverbindung oder Transfizieren der Zellen mit einem Expressionsvektor, die die Exprimierung einer genannten Verbindung im Inneren der genannten Zellen unter Bedingungen ermöglichen, die eine Aktivierung der Exprimierung der Nucleinsäuresequenz durch ein Zytokin zulassen,
(d) Messen der Exprimierung der genannten Nucleinsäuresequenz und
(e) Vergleichen dieser Messung mit der Messung der Exprimierung der genannten einen Nucleinsäuresequenz, die unter den gleichen Versuchsbedingungen, insbesondere der Aktivierung der Exprimierung, in Abwesenheit der genannten Kandidatverbindung beobachtet wird, wobei eine Verringerung der genannten Exprimierung Rückschlüsse auf die Befähigung der genannten Kandidatverbindung zur Inhibierung der durch das Zytokin bewirkten Aktivierung der Exprimierung der Nucleinsäuresequenz zulässt, die für alles oder einen Teil des Polypeptids unter der Steuerung von allem oder einem Teil des Promotors codiert, der das Element GAS: 5'-TTCTGATAAA-3' und die Box E: 5'-CACGTG-3' umfasst.

6. Verfahren gemäß Anspruch 5, **dadurch gekennzeichnet, dass** das Zytokin γ-Interferon ist.

7. Verfahren gemäß Anspruch 5, **dadurch gekennzeichnet, dass** die Zelllinie die Linie Me67.8 ist.

8. Verfahren gemäß einem der Ansprüche 4 bis 7, **dadurch gekennzeichnet, dass** die genannte Nucleinsäuresequenz für alles oder einen Teil des Polypeptids CIITA der Sequenz SEQ ID NO: 2 codiert.

9. Verfahren gemäß einem der Ansprüche 4 bis 7, **dadurch gekennzeichnet, dass** die genannte Nucleinsäuresequenz für alles oder einen Teil eines Reportergens und insbesondere für alles oder einen Teil des Kaninchen-β-Globulins, der Luciferase oder der β-Lactamase codiert.

10. Verfahren gemäß einem der Ansprüche 4 bis 7, **dadurch gekennzeichnet, dass** der genannte Promotor, der das Element GAS: 5'-TTCTGATAAA-3' und die Box E: 5'-CACGTG-3' umfasst, der Promotor IV der Sequenz SEQ ID NO: 1 ist.

11. Verfahren gemäß einem der Ansprüche 4 bis 10, **dadurch gekennzeichnet, dass** die Messung der Exprimierung der genannten Nucleinsäuresequenz auf der Messung spezifischer Messager-RNA's beruht, die ausgehend von der genannten Nucleinsäuresequenz exprimiert werden.

12. Verfahren gemäß einem der Ansprüche 4 bis 10, **dadurch gekennzeichnet, dass** die Messung der Exprimierung der genannten Nucleinsäuresequenz auf der Messung des exprimierten Polypeptids beruht.
